# EUROPEAN PATENT SPECIFICATION

(11) **EP 4 505 605 B1**
(45) Date of publication and mention of the grant of the patent: **02.09.2026**
(21) Application number: 23718176.3
(22) Date of filing: 29.03.2023
(51) Int. Cl.: H03K 17/955, A61M 3/02, G01F 23/263, G01F 23/80

(54) **CAPACITIVE SENSOR ASSEMBLY**
KAPAZITIVE SENSORANORDNUNG
ENSEMBLE CAPTEUR CAPACITIF

(30) Priority: 08.04.2022 DK PA202270195
(43) Date of publication of application: 12.02.2025
(73) Proprietor: Coloplast A/S, 3050 Humlebaek (DK)
(72) Inventor: SALKA, Benjamin Holm, 2200 Copenhagen N (DK); HVID, Niels, 2950 Vedbaek (DK)
(86) International application number: PCT/DK2023/050070
(87) International publication number: WO 2023/193864

(56) References cited:
- EP-A1- 2 918 964
- WO-A1-2009/152568
- US-A- 3 553 575

## Description

The present disclosure relates to a capacitive sensor assembly for measuring one or more capacitive characteristics of a medium in a tubing. In particular, the disclosure relates to a capacitive sensor assembly arranged on a PCB. Further, the disclosure relates to a bowel irrigation system comprising a capacitive sensor assembly according to the disclosure and to a method of manufacturing such an irrigation system.

An irrigation system for bowel irrigation and a method of manufacturing said irrigation system according to the invention are defined in appended claims 1 and 12. Further preferred embodiments are defined in the dependent claims.

### Brief description of the drawings

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Fig. 1 illustrates a perspective view of an exemplary capacitive sensor assembly according to an embodiment of the invention;
Fig. 2 illustrates a top view of an exemplary capacitive sensor assembly according to an embodiment of the invention;
Fig. 3A illustrates a top-view of an exemplary passage embodied as a through-going opening according to an embodiment of the invention;
Fig. 3B illustrates a top-view of an exemplary passage embodied as a through-going opening according to an embodiment of the invention;
Fig. 4 illustrates a perspective view of an exemplary capacitive sensor assembly according to an embodiment of the invention;
Fig. 5 illustrates a perspective view of an exemplary capacitive sensor assembly according to an embodiment of the invention;
Fig. 6 illustrates a perspective view of an exemplary embodiment of a capacitive sensor assembly according to an embodiment of the invention;
Fig. 7 illustrates a top view of an exemplary capacitive sensor assembly according to an embodiment of the invention;
Fig. 8 illustrates a perspective view of an exemplary embodiment of a capacitive sensor assembly according to an embodiment of the invention;
Fig. 9 illustrates a cross-sectional view of a part of an exemplary irrigation system comprising a capacitive sensor assembly according to an embodiment of the invention;
Fig. 10 illustrates a method of manufacturing an irrigation system according to embodiments of the invention;
Fig. 11 illustrates an exemplary build of a capacitive sensor of the capacitive sensor assembly according to embodiments not part of the claimed invention;
Fig. 12 illustrates an exemplary build of a capacitive sensor of the capacitive sensor assembly according to embodiments not part of the claimed invention.

### Background

US 3 553 575 A relates to a sensing device for detecting the position of an object in a sight glass. A pair of capacitor plates is mounted adjacent to a reference position, and the capacitance is affected by the presence of the object.

WO 2009/152568 A1 relates to an apparatus for facilitating irrigation of the bowel. The apparatus comprises a vessel mounted in a solid container. Variable pressure is applied, by an air pump, between the walls of the container and the vessel in order to cause irrigant to be introduced to an intestine via an irrigation member within the flexible bag. A control system is also disclosed, comprising a computational device for controlling the bowel irrigation.

### Detailed description

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

In the following, whenever referring to a medium, such medium may be a solid material or a fluid. In particular, by a medium is meant a substance or physical matter, such as matter through which signals, waves or forces may pass. In the following, whenever referring to a fluid, the referral is to any liquid, gas or other material that continuously deforms under an applied shear stress, or external force, as defined in fluid dynamics. In particular, within the present invention, the term "fluid" includes both the liquid and gas phases. In other words, a fluid may be a liquid or a gas. In the following, whenever referring to a liquid, the referral is to a (nearly) incompressible fluid that conforms to the shape of its container but retains a (nearly) constant volume independent of pressure. In the following, whenever referring to a gas, the referral is to a fluid that has neither independent shape nor volume. In particular, within the present invention, the gas may be (ambient) air, unless otherwise specified. Thus, "gas" and "air" may be used interchangeably.

The present disclosure provides a capacitive sensor assembly for measuring one or more capacitive characteristics of a medium in a tubing, an irrigation system comprising a capacitive sensor assembly and a method of manufacturing an irrigation system comprising a capacitive sensor assembly.

In a first aspect of the invention, a capacitive sensor assembly for measuring one or more capacitive characteristics of a medium in a tubing is disclosed. The capacitive sensor assembly comprises:
- a printed circuit board (PCB) comprising an interior edge defining a passage for the tubing, the interior edge being configured to at least partly abut the tubing;
- a capacitive proximity sensor module;
- a conductive sensor face coupled to the capacitive proximity sensor module; and
- a conductive ground face electrically grounded relative to the capacitive proximity sensor module and arranged facing the sensor face to form a capacitive sensor;
wherein the capacitive proximity sensor module, the sensor face and the ground face are arranged on the PCB and configured to measure the one or more capacitive characteristics of a medium in a tubing arranged in the passage.

In the following, the capacitive sensor assembly is referred to as merely "the assembly".

By a capacitive characteristic is meant a parameter or physical quantity relating to capacitance, or derived from capacitance, such as permittivity. In embodiments, the capacitive characteristic is selected from one or more of (i) a capacitance as induced by a medium, (ii) a permittivity of a medium, (iii) a change of the capacitance as induced by a medium, and (iv) a change of the permittivity. A capacitive characteristic may also be denoted a parameter, such as a capacitive parameter, implying the nature of the parameter being capacitance or associated with capacitance.

Preferably, the assembly is arranged/configured to measure one or more capacitive characteristics of a medium in a tubing. In a preferred embodiment, the assembly is configured to detect presence of liquid or gas in the tubing based on analysis of the one or more capacitive characteristics as measured. For example, the assembly may be configured to detect a change from liquid being present in the tubing (in vicinity of the capacitive sensor and thus causing a certain capacitive characteristic to be measured, such as a first capacitance) to gas being present in the tubing (in vicinity of the capacitive sensor and thus causing another certain capacitive characteristic to be measured, such as a second capacitance different from the first capacitance), and vice versa. The change of the capacitive characteristic being measured (e.g., capacitance) may be detected by the assembly as such, such as via a processor contained in the assembly. Alternatively, the assembly may be configured to merely relay signals to an external processor connected to the assembly, such that said external assembly is configured to perform calculations based on the readings by the assembly.

The assembly comprises a printed circuit board (PCB).

By a PCB is meant a PCB as commonly employed in the field of electronics. For example, by a PCB may be meant a laminated sandwich structure of conductive and insulating layers. The PCB is for affixing electronic components in designated locations and for providing reliable electronic connections between the component's terminals in a controlled manner. For example, the PCB mechanically supports electronic components and may provide for connecting them using traces, planes and other features etched from one or more sheet layers laminated onto and/or between sheet layers of a non-conductive substrate. In the present context, the electronic components include the capacitive proximity sensor module; a conductive sensor face coupled to the capacitive proximity sensor module; and a conductive ground face electrically grounded relative to the capacitive proximity sensor module and arranged facing the sensor face to form a capacitive sensor.

In an embodiment, the PCB is planar and has a first thickness of maximum 3 mm, such as 3 mm, 2 mm, or 1 mm. The PCB may have a non-zero minimum thickness.

The PCB comprises an interior edge defining a passage for a tubing. Thus, it is implied that the PCB further comprises an external edge defining the overall shape/extent of the PCB in an assembly plane. Thus, the PCB defines an assembly plane, and the exterior edge defines the outline of the PCB in such assembly plane.

By defining a passage for a tubing is meant that the PCB comprises a through-going passage, such that a tubing may pass through the PCB. In embodiments, the passage is closed, such that the interior edge is not connected to the exterior edge. Such a closed passage may be denoted a through-going opening or an aperture in the PCB. Thus, in an embodiment, the passage is a through-going opening, or aperture, in the PCB.

In embodiments, the passage is open, such that the interior edge is connected to the exterior edge. In such embodiment, the exterior edge may be said to comprise an indentation or notch in the assembly plane, the edge(s) of such indentation or notch thus being referred to as the interior edge. Thus, in an embodiment, the passage is a notch extending from an exterior edge of the PCB and wherein the interior edge is connected to the exterior edge.

The interior edge is configured to at least partly abut the tubing. By at least partly abut is meant that the tubing, and in particular an outer surface thereof, and the interior edge may, in embodiments, in at least one point, touch each other. For example, in embodiments where the passage is open, the interior edge may not entirely encircle or surround the tubing, but may in sections thereof, touch the tubing.

In embodiments, the interior edge is adapted to an outer geometry of the tubing such that the interior edge touches, or is in close proximity or vicinity of, the outer surface of the tubing, whereby the interior edge and the tubing may be said to abut each other. For example, where the tubing is circular in cross-section and has a diameter of 10 mm, the interior edge may define a circular passage having an internal diameter of at least 10 mm. However, in order to increase the capacitive response from the sensor, the internal diameter is preferably not substantially greater than the diameter of the tubing as such. For example, the internal diameter may be less than 50 % greater than the diameter of the tubing to be used with the assembly. Thereby, if the tubing has a diameter of 10 mm, the internal diameter may be between 10 mm and 15 mm. In embodiments, the internal diameter is less than 10 %, or less than 20 %, less than 30 %, or less than 40 %, or less than 75 %, or less than 100 % greater than the diameter of the tubing to be used with the assembly. Thus, by abut may also mean to be in close proximity of each other, where close proximity may be defined by the above percentages. Finally, it should be noted that the tubing may be a (rigid) channel provided in an element, and in such embodiments, the interior edge may be configured to abut an outer surface of such channel. Being a non-invasive measurement technique, the capacitive sensor does not require immediate and physical contact, and as such, by abut is also meant to be in such proximity of the sensor and ground face that a capacitive characteristic, such as capacitance, of a medium in the tubing may be reliably and repeatably measured.

Instead of defining a circular passage, the interior edge may define a rectangular passage in the assembly plane. The rectangular passage may be defined by a closed interior edge, or the rectangular passage may be defined by an open interior edge, in which case the rectangular passage may be a rectangular indentation or notch extending from the exterior edge in the assembly plane. In case of a rectangular passage, the relevant dimension will be a side length of the rectangle, such that said side length is to be greater than the diameter of the tubing, which side length may further have the above-stated dimensional restrictions.

The assembly comprises a capacitive proximity sensor module; in the following referred to as the module. The sensor face is coupled/connected to the module. The module, in combination with the sensor face, forms a (capacitive) proximity sensor. The module may comprise a first processor. The first processor may be configured to generate a binary output indicative of the one or more capacitive characteristics of the medium in the tubing. For example, the first processor may provide a binary output in accordance with a monitored capacitive characteristic being above or below a set threshold.

By a capacitive proximity sensor is meant a sensor type suited to detect presence of nearby objects/materials without any physical contact. In particular, a capacitive proximity sensor utilizes the change of capacitance based on a change in the electrical field around an active face (in the following also denoted a sensor face) of the sensor. The target/object to be sensed will act as the second plate/face of a plate capacitor thus formed between the active face of the sensor and said target. In other words, a capacitive proximity sensor may thus work without a second fixated plate and yet be modelled according to a plate capacitor, as the target to be sensed will act as the second plate. Typically, the proximity sensor comprises, such as in the module, an internal oscillator circuit such that, as the target approaches the active face, oscillations increase until they reach a threshold level and activate/generate an output, such as a binary output. The threshold level may be adjusted according to the desired use of the proximity sensor. In other words, by a capacitive proximity sensor module is meant a module comprising basic computational capabilities sufficient to perform measurements of a capacitive characteristic, such as capacitance and/or permittivity based on capacitance. Alternatively, the module may be configured to determine whether the capacitive characteristic is above or below one or more thresholds, but not necessarily capable of determining an absolute value of the characteristic. For example, the module may be configured to generate a binary output based on whether the capacitive characteristic considered is above or below a set threshold. For example, the threshold may be set according to a desired target: for example, where the assembly is for determining whether liquid (e.g., water, having a relative permittivity of approximately 80) or gas (e.g., air, having a relative permittivity of approximately 1) is present in the tubing, the threshold may be set accordingly to differentiate between such relative permittivities.

The module may be a 6-pin single-channel proximity detector. The sensor may have an operative voltage in the range from 2.0 V to 5.5 V. The sensitivity (thresholds) may be set via one or more of the six pins.

A proximity sensor may also be denoted a touch sensor or a distance sensor in the field.

The first processor of the module may comprise a hysteresis function, such that data as measured by the capacitive sensor may be filtered before further processing, such as further processing by a processor coupled to the assembly. The hysteresis function may reduce fluctuations in the binary output of the first processor, such as fluctuations as caused by insignificant irregularities in the medium flowing through the tubing, such as air bubbles in a stream of liquid passing by the capacitive sensor (e.g., in the tubing). Further, the hysteresis function may filter out minor external disturbances, such as disturbances caused by external objects in the vicinity of the capacitive sensor, such objects potentially disturbing the electric field of the capacitive sensor.

In embodiments, the hysteresis function may be embodied by the use of different thresholds dependent on the direction of change of the capacitive characteristic being monitored (e.g., capacitance). For example, if an input signal (e.g., the measured value of capacitance) to the hysteresis function/algorithm is higher than an upper threshold value, the binary output is an upper output value (e.g., "1" indicative of liquid) and if the input signal is lower than a lower threshold value, the binary output is a lower output value (then, "0" indicative of gas), wherein the upper threshold value is greater than the lower threshold value. Thus, between the lower and upper threshold values, the binary output may be maintained according to the most-recent binary output.

In embodiments, the hysteresis function may have a maximum hysteresis of 10 %, or of 20 %, or of 30 %, or of 50 %. In embodiments, the hysteresis function may have a maximum hysteresis of at least 10 %, or of at least 20 %, or of at least 30 %, or of at least 40 %, or of at least 50 %. Stating a maximum hysteresis in percentage may be understood such that, when a standardised target is within X mm of the sensor, the sensor may generate an output, and for the output to change, the target must be moved +Y % of the X mm. For example, where a target within 10 mm of the sensor causes a change in output, the hysteresis function may be adapted to change that output if the object is moved more than 10 % away (i.e., in this example, if the target is moved to 11 mm or more).

Further, in embodiments, the first processor may consider both its input at a given time and its past behaviour. For example, the past behaviour may include inputs for the past (trailing) 1 s, or for the past 5 s, or for the past 10 s, or for between 10 s and 60 s, such as up to 60 s. Thereby, at a given time, if the capacitive characteristic was, on average, indicative of liquid in the past (for example) 10 s, a deviation therefrom (such as presence of gas) would not cause a change in the binary output.

The use of a hysteresis function allows for a continuous/uninterrupted operation of the device incorporating the assembly, such as where the one or more capacitive characteristics are used to control the device. Thus, the hysteresis function may be designed to, or considered to, purposively lower the sensitivity of the capacitive sensor.

The assembly comprises an electrically conductive sensor face coupled to the module, thereby forming a proximity sensor as discussed above. The sensor face is electrically conductive to function as the first plate of a capacitor.

The assembly may comprise a conductive ground face electrically grounded relative to the capacitive proximity sensor module and arranged facing the sensor face to form a two-plate capacitor. For example, the ground face is a floating ground, such as wired to a ground (negative) of a battery for powering the module.

In alternative embodiments, the assembly does not comprise a ground face. In such embodiments, the capacitive sensor may be considered a proximity or distance sensor, and whereas it may be more prone to external disturbances, it may be shielded by other means, such as by means of shielding in a device incorporating the assembly. It is appreciated that embodiments of the assembly as disclosed herein may all be fitted with a capacitive sensor without a ground face - such as by merely omitting the provision of the ground face from the embodiments disclosed. For example, embodiments comprising a reference to the shape or arrangement of the sensor face may be applicable to a capacitive sensor merely comprising the sensor face. However, for the majority of the present disclosure, embodiments comprising both a sensor face and a ground face are discussed.

In embodiments, the sensor face and ground face are provided on the interior edge, such as where the interior edge as such defines the sensor and ground face, as will be discussed in greater detail below. In embodiments, the sensor face and ground face are provided as conductive plates coupled to, such as soldered to, the PCB, such as coupled to the interior edge in two parts. For example, each of the plates may have a surface area being considerably bigger than a surface area of the interior edge, or a surface area of the part of the interior edge each of the plates cover, such as twice as big, or such as five times as big.

The sensor face and the ground face together form a two-plate capacitor, thus implying that the faces are separated by a distance. The plates may be parallel, to form a parallel-plate capacitor, or the plates may be non-parallel, such as forming a semi-cylindrical capacitor where each face is fitted/adapted to the outer surface geometry of a circular tubing. For example, where the tubing is circular, the faces may be curved to fit about parts of the tubing. Fitting the faces according to the tubing may increase the sensitivity of the capacitor, or increase the signal, or reduce the noise.

The capacitive proximity sensor module, the sensor face, and the ground face are arranged on the PCB. For example, the module may be soldered onto the PCB and a wiring may be formed between the module and the sensor face, as these are coupled as previously disclosed. Further, the ground face may be provided with a wiring extending to a ground wiring.

The capacitive proximity sensor module, the sensor face, and the ground face are configured to measure the one or more capacitive characteristics of a medium in a tubing arranged in the passage. In other words, in particular the sensor face and the ground face are arranged on the PCB such that, when a tubing is arranged in the passage, said tubing is arranged between said faces forming a two-plate capacitor. For example, the sensor face may be arranged on a first side of the passage and the ground face may be arranged on the second side of the passage, such that the tubing extends between said faces. Preferably, the faces are arranged perpendicular to the assembly plane, such that they are flush with the tubing when said tubing extends perpendicularly through the passage of the PCB. In preferred embodiments, the faces are arranged in parts of the interior edge, as will be discussed further below.

Generally, by a capacitive sensor, such as the sensor formed by the module, the sensor face, and the ground face of the present disclosure, is meant a sensor configured to measure a capacitance or derivative thereof. In other words, the capacitive sensor is configured to measure one or more capacitive characteristics of a dielectric medium (e.g., a fluid or solid matter) in proximity to the capacitive sensor, such as between one or two elements constituting the faces/plates of a two-plate capacitor. Thus, in embodiments, the capacitive sensor comprises a capacitor arranged to measure one or more capacitive characteristics of a tubing and a medium therein. By a capacitive characteristic is meant any electrical quantity or value associated with, or derivable from, a capacitor, including the absolute value of capacitance, a change of capacitance over time, the absolute value of the (relative) permittivity of a fluid, or a change of (relative) permittivity (e.g., as caused by a change in the dielectric medium in proximity to the capacitive sensor over time). The capacitive sensor, such as the module, may be configured to continuously monitor the one or more capacitive characteristics of the tubing, such as at a frequency higher than 0.1 Hz, such as higher than 1 Hz, such as higher than 10 Hz or higher than 100 Hz.

In general, the capacitance C of a parallel-plate capacitor may be given as: $C = ε \frac{A}{d} = {ε}_{r} {ε}_{0} \frac{A}{d}$ wherein *ε* is the permittivity of the (dielectric) medium between the parallel plates, A is the area of each of the parallel plates, and d is the separation of the parallel plates, said separation being a gap filled with the (dielectric) medium. The permittivity *ε* may be expressed as the product of the relative permittivity *ε*ᵣ of the medium and the vacuum permittivity *ε*₀. As such, a medium having a permittivity ε may be said to induce a capacitance when the area A and the separation d are constant. The vacuum permittivity *ε*₀ is a physical constant having the value 8.8541878128 × 10⁻¹² F m⁻¹, and the relative permittivity *ε*ᵣ of water is 80.2 (at 20 °C) and the relative permittivity of air is 1.00058986. For vacuum, the relative permittivity is one by definition. Thus, the relative permittivity of water is approximately 80 times the relative permittivity of air, which translates to a similar change of the size of the capacitance of the capacitive sensor when a change from liquid to air occurs in the tubing in proximity to the capacitive sensor and vice versa.

Whereas embodiments of the capacitive sensor of the present invention may deviate from that of a parallel-plate capacitor, the theory of the parallel-plate capacitor is here used to highlight the overall relationship between permittivity (material dependent) and geometrical factors of the build of the capacitive sensor (area and separation). Thus, the present discussion refers to the above relationship for the capacitance of a parallel-plate capacitor, and it is appreciated that the discussion may be applied to other builds of capacitors wherein the capacitance may be calculated differently according to the geometry of such different builds. In other words, whereas not all embodiments of the capacitive sensor according to the present invention may be modelled as a parallel-plate capacitor, the model may be used to express the general relation between capacitance, permittivity, and geometry of the capacitor.

In embodiments, when a medium passes through a plate capacitor (e.g., through a tubing arranged between the faces of the capacitor), the capacitance is dependent on (is induced by) the permittivity of said medium. For example, when the medium is water, the capacitance is dependent on the relative permittivity for water, and when the medium is air, the capacitance is dependent on the relative permittivity for air. The relative permittivity of water is approximately 80 times the relative permittivity of air, and as such, the change of capacitance is significant when the characteristics (e.g., the presence of air bubbles in the flow of water) of the medium changes over time. The permittivity of the material (e.g., plastic) of the tubing as such, arranged in vicinity of the capacitor, may be neglected as it may be fixated relative to the capacitive sensor, and thus does not vary over time.

In other words, the capacitance over time C(t) is dependent on the changes of permittivity (of the fluid in the tubing) over time ε(t) in the capacitor: $C \left(t\right) = ε \left(t\right) \frac{A}{d} = {ε}_{r} \left(t\right) {ε}_{0} \frac{A}{d}$

The assembly according to embodiments of the present disclosure is configured to monitor the time-dependent capacitive characteristics, such that the assembly may monitor changes in the capacitive characteristic and preferably generate a signal, such as a binary signal, indicative of such change, such as a change from the first capacitance to a second capacitance different from the first capacitance.

In a preferred embodiment, the assembly comprises an assembly interface configured to connect the module, and potentially the ground, to a target device. By a target device is meant any device to which the assembly may be coupled. In particular, the target device may be a device comprising the tubing, and where it is desired to monitor such tubing (e.g., the contents thereof) by means of the assembly. Thereby, the target device may be adapted to be fitted with the assembly and connect to the assembly via the assembly interface. For example, where the target device comprises the tubing, the assembly may be arranged such that the tubing extends through the passage of the assembly, and such that the target device, via the assembly, may monitor the contents of the tubing by means of analysing the capacitive characteristics.

In a preferred embodiment, the assembly is powered by a power source of the target device via the assembly interface. Thereby, the assembly does not need a separate power source to be arranged on the PCB. In an alternative embodiment, the assembly comprises a power source, such as a battery, arranged on the PCB and coupled to the module, and where the ground is grounded relative to a ground terminal of the power source.

Providing the capacitive sensing capabilities in an assembly as disclosed provides for an optimised manufacturing of a target device configured to be fitted with the assembly. Namely, the provision of an assembly as disclosed allows for a parallel manufacturing, and distribution, of the assembly, such that the manufacturing of the target device may merely include a step of inserting the assembly, rather than building a capacitive sensor from scratch in the target device as such. Thereby, the manufacturing speed of the target device may be considerably increased.

Further, an assembly as disclosed allows for easy adaption to the desired use: the assembly may be adapted to a wide range of applications, since all the electronics is provided in a single PCB, which may be easily installed in a target device.

The ride range of applications may include, but is not limited to, monitoring of gas pipes, monitoring of water pipes, monitoring of pipes in a manufacturing facility where objects are passing through a tubing, and where it is desired to monitor the speed, size, number or the like of such objects. For example, the objects may be counted by means of analysing the change of capacitance or permittivity over time, as the passing of an object alters the capacitance if the permittivity of the object is different from, e.g., the ambient air in the tubing. Further, the assembly may be included in medical devices wherein fluids are flowing, such as where it is desired to know whether liquid or gas is present in the tubing, such as in dialysis, during infusion, catheterization, or bowel irrigation. In a second aspect of the invention, a bowel irrigation system comprising an assembly according to the first aspect of the invention is disclosed. Thus, whereas specific embodiments of a target device are disclosed, it is appreciated that the assembly according to the first aspect of the invention may be utilized or incorporated in a wide range of target devices wherein it is desired to monitor a tubing.

Finally, the provision of a capacitive sensor allows for analysing a medium in the tubing non-invasively. Namely, a capacitive sensor, by means of the underlying physics of capacitance, is inherently a proximity sensor, such that a face of the sensor need not to be in physical contact/interaction with the medium to be analysed/measured. Thus, assembly is suitable for incorporation in the wide range of target devices, as the assembly does not require physical interaction with the medium of the tubing. Thereby, the tubing need no special adaptions for functioning with the assembly: the assembly may merely need to be adapted to the size and geometry of the tubing.

In an embodiment, the interior edge is electrically conducting in a first part and in a second part separate from the first part. The first and second part may be electrically conducting by means of being plated, coated, or covered with an electrically conducting material, such as a metal (e.g., copper, silver, etc.) or a carbonaceous material (e.g., carbon).

In an embodiment, the first part of the interior edge forms the sensor face, and the second part of the interior edge forms the ground face. Thereby, the sensor and ground faces are defined by the conductive first part and second part of the interior edge. In other words, the sensor face and the ground face are provided or formed in the interior edge of the passage of the PCB, whereby the sensor face and ground face have an extension in the direction perpendicular/normal to the assembly plane or parallel with the extension of the thickness of the PCB. Preferably, the sensor face and the ground face cover the entire thickness of the PCB. In other words, where the PCB has a certain thickness (e.g., 3 mm), the sensor face and ground face have an extension of the same length (here, 3 mm) in the direction normal to the assembly plane. In further other words, in embodiments, the sensor face and the ground face cover the entire thickness/height of the interior edge. Accordingly, in embodiments, a surface area of the sensor face may be defined by a length of the first part, such as a length of the corresponding arc, and a thickness of the PCB. Likewise, a surface area of the ground face may be defined by a length of the second part, such as a length of the corresponding arc, and a thickness of the PCB.

Thereby, the faces may immediately abut a tubing, when such tubing is arranged in a passage adapted to the tubing - the tubing likewise extending in the direction normal to the assembly plane, such that the faces are parallel with the extension of the tubing through the passage.

In embodiments, the passage is a through-going opening in the PCB, thus being an aperture in the PCB having a continuous interior edge. In an embodiment thereof, the through-going opening is circular. Thereby, the through-going opening, and thus the assembly, is adapted for a tubing having a like circular cross-section, such that said tubing may extend through the opening. In embodiments, the circular opening is connected to the exterior edge via a slit in the PCB.

In embodiments, the through-going opening has a diameter, or maximum dimension in the assembly plane (if not circular), between 5 mm and 200 mm. The size of the range indicates that the assembly may be used in a wide range of applications. However, in embodiments of further aspects of the invention, the diameter is preferably in the range from 5 mm to 50 mm, such as from 5 mm to 30 mm, such as from 8 mm to 20 mm.

In embodiments, where the through-going opening defines a circle, the first part may be arranged in a first arc of the circle and the second part may be arranged in a second arc of the circle, the arcs being separate/non-overlapping. In embodiments, the first arc is less than 180 degrees, and the second arc is less than 180 degrees. In embodiments, the first and the second arc are between 45 degrees and 180 degrees each, preferably between 45 degrees and 170 degrees each, or between 90 degrees and 135 degrees each, such as 90 degrees each. In embodiments, the first and the second arc are less than 45 degrees each. In an embodiment, each of the first part and the second part of the interior edge are configured to abut the tubing in an arc less than 170 degrees each, thereby providing a free space of 10 degrees (e.g., two times 5 degrees) between end-portions of each first and second part.

Accordingly, the first and second parts may be said to form semicylindrical faces defined by the thickness of the PCB and the length of the corresponding arc as discussed above. Thus, in an embodiment, the sensor face and the ground face are semicylindrical faces on the interior edge. The arcs are arranged diametrically opposite, such that the first and second parts face each other in the opening/passage.

Providing an arc of less than 180 degrees, in particular less than 135 degrees, such as 90 degrees, increases an effective separation distance of the two-plate capacitor and reduces the electric field contributions caused by end-portions of each curved plate being in closer proximity than the rest of the plates when the first and second part are configured to constitute the sensor face and the ground face, as discussed above.

As previously introduced, in embodiments, the passage is open, such that the interior edge is connected to the exterior edge. In the latter embodiment, the exterior edge may be said to comprise an indentation or notch in the assembly plane, the edge(s) of such indentation or notch thus being referred to as the interior edge. Thus, in embodiments, the passage is a notch extending from an exterior edge of the PCB and wherein the interior edge is connected to the exterior edge. In an embodiment thereof, the interior edge comprises three interior sub-edges configured to at least partly abut the tubing, the three interior sub-edges comprising a first interior sub-edge and a second interior sub-edge, the second interior sub-edge being arranged in parallel with, and separate from, the first interior sub-edge. In an embodiment thereof, the first interior sub-edge is conductive and forms, or defines, the sensor face and the second interior sub-edge is conductive and forms, or defines, the ground face. The first and second sub-edges may be at least partly conductive, such as not necessarily in their entire extent.

A notch/indentation in the PCB as defined here may resemble a rectangular notch, where a fourth edge may be defined as the opening in the assembly plane, such that the tubing may be inserted into the passage by means of translating the tubing in the assembly plane. On the contrary, when the passage is an opening/aperture without access in the assembly plane, the tubing must be threaded through the opening/aperture.

The notch as defined according to embodiments herein may receive a tubing having an arbitrary cross-section, including a circular cross-section and a rectangular cross-section, as long a maximum dimension of the cross-section of the tubing may fit into notch.

The notch as defined according to embodiments herein may be provided with the sensor face and ground face on opposite sides. Namely, the sensor face may be defined/formed on the first interior sub-edge, and the ground face may be defined/formed on the second interior sub-edge, where said first and second sub-edges are opposite and facing each other. Accordingly, in such embodiments where the sub-edges are parallel, a parallel-plate capacitor is formed by the sensor face and the ground face, and the tubing may pass through the capacitor by means of passing through the notch.

In embodiments, the notch has a tube maximum dimension in the assembly plane between 5 mm and 200 mm. By a tube maximum dimension is meant the maximum dimension of a tube the notch may receive, such that the capacitive sensor may measure one or more capacitive characteristics of a medium in the tubing. The size of the range indicates that the assembly may be used in a wide range of applications. However, in embodiments of further aspects of the invention, the diameter is preferably in the range from 5 mm to 50 mm, such as from 5 mm to 30 mm, such as from 8 mm to 20 mm.

In a second aspect of the invention, an irrigation system for bowel irrigation comprising a capacitive sensor assembly according to the first aspect of the invention is disclosed.

In the following, whenever referring to a bowel irrigation system or bowel irrigation, the referral to a system or method capable of irrigating the bowels of a user using a catheter. Commonly, the catheter is inserted through the anus. Bowel irrigation (systems) is also known in the art as anal irrigation (systems) and rectal irrigation (systems), and the terms may be used interchangeably in embodiments where the bowel irrigation system is adapted for use through the anus.

In the following, whenever referring to the bowel(s) of a user, the referral is to the intestines of the user. The referral can be to the lower intestines specifically, e.g., the rectum and/or the colon/large intestine. In the following, whenever referring to the rectum, the referral is to the terminal section/canal of the intestine ending in the anus. In the following, whenever referring to the anus, the referral is to the opening of the lower end of the alimentary canal, through which refuse of digestion is commonly excreted. In the following, whenever referring to anal, the referral is to a feature, device, method, or system pertaining to the anus, e.g., pertaining to engagement with or through the anus. In the following, whenever referring to the rectal walls, the referral is to the intestinal wall surrounding and defining the canal of the rectum.

Bowel irrigation is one of a number of treatments used to aid people with bowel problems. People suffering from bowel problems are often paralyzed, typically due to spinal cord injuries, and confined to a wheelchair or hospitalized. In these situations, often the peristaltic functions, i.e. the reflexes and muscles of the bowel, cannot be stimulated correctly. This results in constipation or random discharge of bowel contents. By using bowel irrigation, a stimulation of the peristaltic movements of the colon can be provided. To perform such bowel irrigation, a device comprising a catheter, also referred to as an anal catheter, anal probe, rectal catheter, or speculum, is provided. The catheter is inserted into the rectum through the anus. A liquid, also referred to as an irrigation liquid, such as water or a saline solution, is then introduced into the rectum/bowels through the catheter. The amount of liquid is generally up to 1.5 litres, depending on the person. The introduced liquid stimulates the peristaltic movements of the bowel. After a specified period of time, such as 15 minutes, the catheter is removed, and the liquid, along with output from the bowel, is released through the anus.

The irrigation system according to the second aspect of the invention may further comprise:
- a housing comprising a reservoir for containing an irrigation liquid;
- a tubing providing fluid communication between the reservoir and a catheter for use with the irrigation system;
- a control unit comprising a processor coupled to the capacitive sensor assembly; and
- a pump for facilitating a flow of irrigation liquid from the reservoir, through the tubing, to the catheter;
wherein the tubing is arranged in the passage of the PCB of the capacitive sensor assembly such that the capacitive sensor assembly is configured to measure one or more capacitive characteristics of a fluid in the tubing.

The irrigation system further comprises a power source, such as a battery, configured to power the pump, the processor, and the assembly. The assembly may be connected to the processor and the power source via the assembly interface as previously disclosed.

The irrigation system as disclosed may be considered a target device as previously defined in relation to the first aspect, and thus provides a specific embodiment of such a target device. It is appreciated that the assembly may be incorporated in other target devices as disclosed.

In embodiments, the processor receives the one or more capacitive characteristics. In embodiments, the processor is configured to control the irrigation system, such as to control an irrigation procedure running in the irrigation system, such as by controlling the pump of the irrigation system, in accordance with the one or more capacitive characteristics.

In a preferred embodiment of the second aspect of the invention, the capacitive sensor of the assembly is configured to detect presence of liquid and/or gas in the tubing of the irrigation system based on analysis of the one or more capacitive characteristics as measured. For example, the capacitive sensor may be configured to detect a change from liquid being present in the tubing (in vicinity of the capacitive sensor and thus causing a certain capacitive characteristic to be measured, such as a first capacitance) to gas being present in the tubing (in vicinity of the capacitive sensor and thus causing another certain capacitive characteristic to be measured, such as a second capacitance difference from the first capacitance), and vice versa. Thus, the capacitive sensor may be specialised in distinguishing/differentiating specifically liquids, such as irrigation liquid, such as water, from gasses, such as ambient air. Thereby, the capacitive sensor may be specifically adapted for use with an irrigation system where liquid and gas are the primary materials flowing in the tubing.

By control is meant that the processor may be configured to influence, regulate, or affect the irrigation procedure in accordance with the measured one or more capacitive characteristics. For example, in other words, the processor may take a capacitive characteristic as input and generate an output comprising instructions on how to operate the pump based on said capacitive characteristic. For example, the output may comprise instructions to power off the pump in accordance with/based on the measured capacitive characteristic. To control may comprise to terminate, initiate, pause, resume/reinitiate the irrigation procedure or to disable irrigation system as such.

In embodiments, the processor is configured to control the irrigation procedure, such as to terminate the irrigation procedure, such as by means of turning the pump off, in response to the one or more capacitive characteristics of the fluid in the tubing being indicative of air In embodiments, the processor is configured to control the irrigation procedure, such as to (re-) initiate the irrigation procedure, such as by means of turning on the pump, in response to the one or more capacitive characteristics of the fluid in the tubing being indicative of liquid, such as irrigation liquid.

In a third aspect of the invention, a method of manufacturing an irrigation system according to the second aspect of the invention is disclosed. The method may comprise the steps of:
- providing the housing, the tubing, the control unit and the pump;
- providing the capacitive sensor assembly;
- assembling the housing, the control unit, the pump and the tubing; and
- arranging the capacitive sensor assembly (of the first aspect of the invention) relative to the tubing such that the tubing passes through the passage of the capacitive sensor assembly.

The method highlights a benefit of the capacitive sensor assembly of the first aspect of the invention: namely, the capacitive sensor assembly may be provided as one-piece in the manufacturing line of the irrigation system. Thereby, the irrigation system may be manufactured through providing the individual pieces and assembling the pieces, wherein the step of arranging the capacitive sensor assembly relative to the tubing involves arranging the assembly such that it is configured to measure one or more capacitive characteristics of a fluid in the tubing. This is achieved by means of arranging the tubing in the passage of the assembly. Namely, the sensor face and the ground face are configured, and thus arranged, to measure the one or more characteristics of a medium (here, fluid) in the tubing when arranged in the passage.

In a general embodiment of a method of manufacturing a target device comprising a capacitive sensor assembly according to the first aspect of the invention, the method may comprise the steps of:
- providing one or more elements of the target device, the one or more elements comprising a tubing;
- providing the capacitive sensor assembly;
- assembling the elements of the target device;
- arranging the capacitive sensor assembly relative to the tubing such that the tubing passes through the passage of the capacitive sensor assembly-

### Detailed description of the drawings

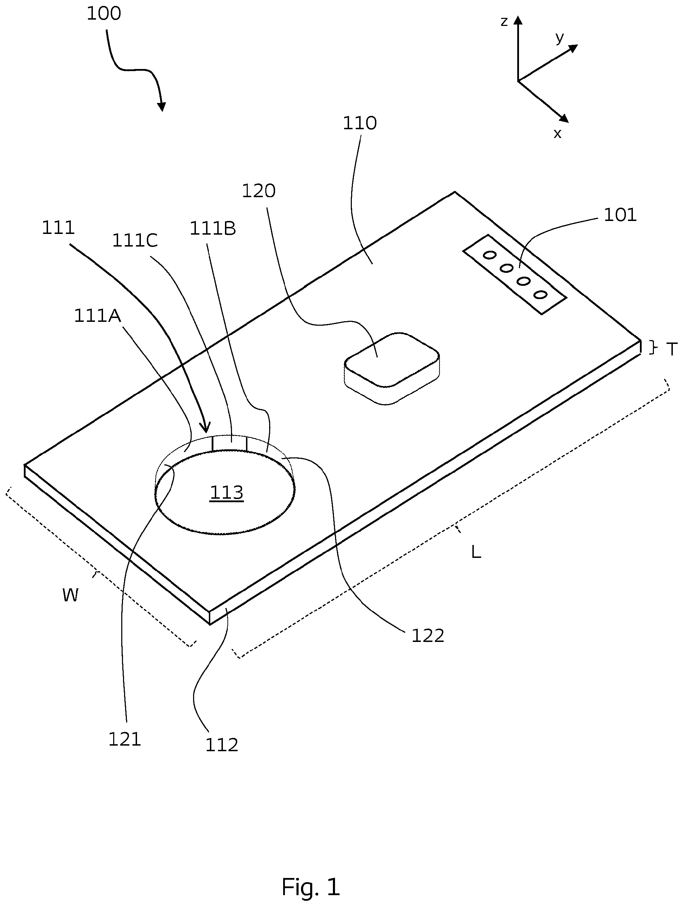

Fig. 1 illustrates a perspective view of an exemplary capacitive sensor assembly 100 according to an embodiment of the invention. The assembly 100 comprises a printed circuit board (PCB) 110 comprising an interior edge 111 defining a passage 113 for a tubing, the interior edge 111 being configured to at least partly abut such a tubing, such as by means of having a size optimised for the intended tubing to be used with the assembly 100. The passage 113 in Fig. 1 is embodied as a through-going opening, or aperture. The PCB 110 further comprises an exterior edge 112. The PCB 110 defines an assembly plane spanning the (x,y)-plane of the indicated coordinate system (x,y,z). The exterior edge 112 defines the overall shape of the PCB 110 in the assembly plane.

Preferably, the PCB is planar and has a first thickness T (i.e., an extension in the indicated z-direction) of maximum 3 mm, such as 3 mm, 2 mm, or 1 mm. The PCB may be rectangular and have a length L (i.e., an extension in the indicated y-direction) in the range from 30 mm to 100 mm, such as from 40 mm to 80 mm, and a width W (i.e., an extension in the indicated x-direction) in the range from 30 mm to 100 mm, such as from 40 mm to 80 mm.

The interior edge 111 is electrically conducting in a first part 111A and a second part 111B separate from the first part 111A. The first part 111A and the second part 111B may be separated by a non-conducting part 111C (a corresponding non-conducting part opposite the part 111C is not shown due to the perspective view). The first 111A and second parts 111B may be electrically conducting by means of being plated, coated, or covered with an electrically conducting material, such as a metal (e.g., copper, silver, etc.) or a carbonaceous material (e.g., carbon).

The first part 111A of the interior edge 111 forms a sensor face 121 and the second part 111B of the interior edge 111 forms a ground face 122. In other words, the assembly 100 comprises a conductive sensor face 121 and a conductive ground face 122, wherein, in the illustrated embodiment, the sensor face 121 and ground face 122 are defined or formed on respective parts of the interior edge 111. Thereby, the sensor face 121 and ground face 122 have an extension in the z-direction preferably equal to the thickness T of the PCB, such that the sensor face and ground face each has a surface area defined by a length (e.g., length in the assembly plane, such as a length of an arc of the circular passage) of the first part and second part, respectively, and a thickness T of the PCB.

The sensor face 121 is coupled to a capacitive proximity sensor module 120 arranged on the PCB 110 (tracing not shown). The ground face 122 is electrically grounded relative to the module 110 and thus the sensor face 121.

The ground face 122 is facing the sensor face 121 to form a two-plate capacitor.

The sensor face 121, the ground face 122, and the module 120 are arranged on the PCB.

In preferred embodiment, the assembly 100 comprises an assembly interface 101 for connection to a target device incorporating the assembly 100. Preferably, the electronics of the assembly 100 is controlled and/or powered by the target device via the assembly interface 101. The assembly interface 101 may comprise one or more terminals for connecting with corresponding terminals of an interface of the target device. For example, the assembly 100 may be connected via the assembly interface 101 to a processor of the target device, said processor receiving measurements of one or more capacitive characteristics from the module 120 connected to the sensor face 121. Thus, preferably, the module 120 is connected to the assembly interface 101. The ground face 122 may be wired to a ground terminal of the assembly interface 101.

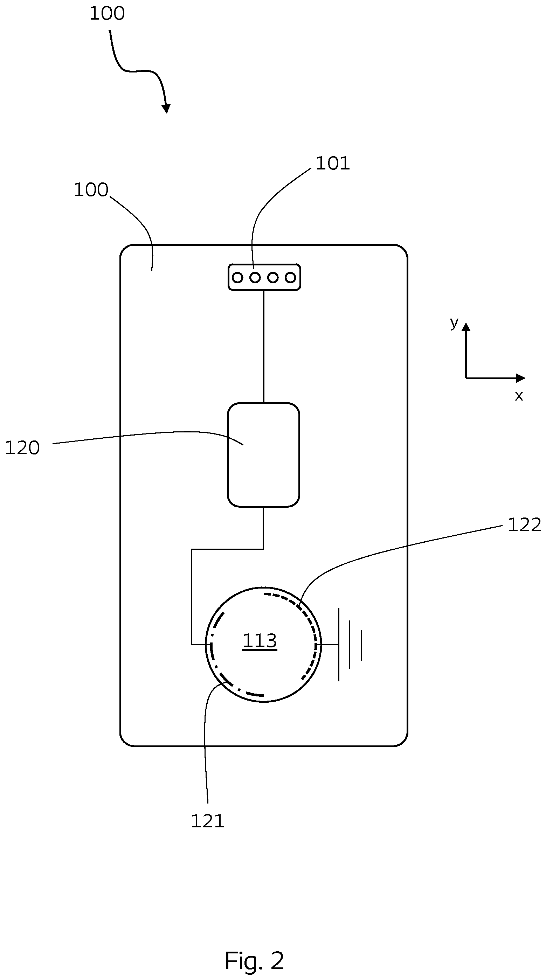

Fig. 2 illustrates a top view of an exemplary capacitive sensor assembly 100 according to an embodiment of the invention, such as the assembly 100 of Fig. 1.

In the illustration, the sensor face 121 is indicated by means of a dashed-dotted arc of a circle and the ground face 122 is indicated by means of a dashed arc of a circle, and it is to be understood that the sensor face 121 and ground face 122 are thus arranged on respective parts, defined by the extent of the arc, of the interior edge of the PCB 100 defining the passage 113. In the illustrated embodiment, each arc is approximately 135 degrees, separated by two non-conducting arcs (not illustrated by a line object) of 45 degrees each.

The sensor face 121 may be connected to the module 120, which is further connected to the assembly interface 101, and the ground face 122 is grounded. For example, the ground face 122 may be grounded by means of a connection to a ground terminal of the assembly interface 101.

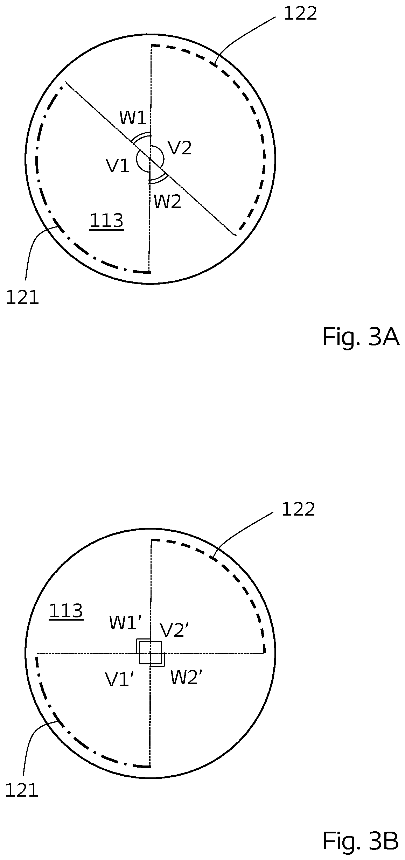

Fig. 3A illustrates a top-view of an exemplary passage 113 embodied as a through-going opening according to an embodiment of the invention. The sensor face 121 is indicated by means of a dashed-dotted arc of a circle and the ground face 122 is indicated by means of a dashed arc of a circle, and it is to be understood that the sensor face 121 and ground face 122 are thus arranged on respective parts, defined by the extent of the respective arc of the interior edge of the PCB 100 defining the passage 113.

In the illustrated embodiment, the sensor face 121 spans an angle V1 of 135 degrees and the ground face 122 spans an angle V2 of 135 degrees, wherein the sensor face 121 and the ground face 122 are separated by two non-conducting parts each spanning an angle W1,W2 of 45 degrees. Thereby, the ground face 122 is arranged oppositely and facing the sensor face 121 to form a two-plate capacitor.

Radii are illustrated by small-dashed lines to highlight the angles.

Fig. 3B illustrate a top-view of an exemplary passage 113 embodied as a through-going opening according to an embodiment of the invention. The sensor face 121 is indicated by means of a dashed-dotted arc of a circle and the ground face 122 is indicated by means of a dashed arc of a circle, and it is to be understood that the sensor face 121 and ground face 122 are thus arranged on respective parts, defined by the extent of the respective arc of the interior edge of the PCB 100 defining the passage 113.

In the illustrated embodiment, the sensor face 121 spans a right angle V1' of 90 degrees and the ground face 122 spans a right angle V2' of 90 degrees, wherein the sensor face 121 and the ground face 122 are separated by two non-conducting parts each spanning a right angle W1',W2' of 90 degrees. Thereby, the ground face 122 is arranged oppositely and facing the sensor face 121 to form a two-plate capacitor.

Radii are illustrated by small-dashed lines to highlight the angles.

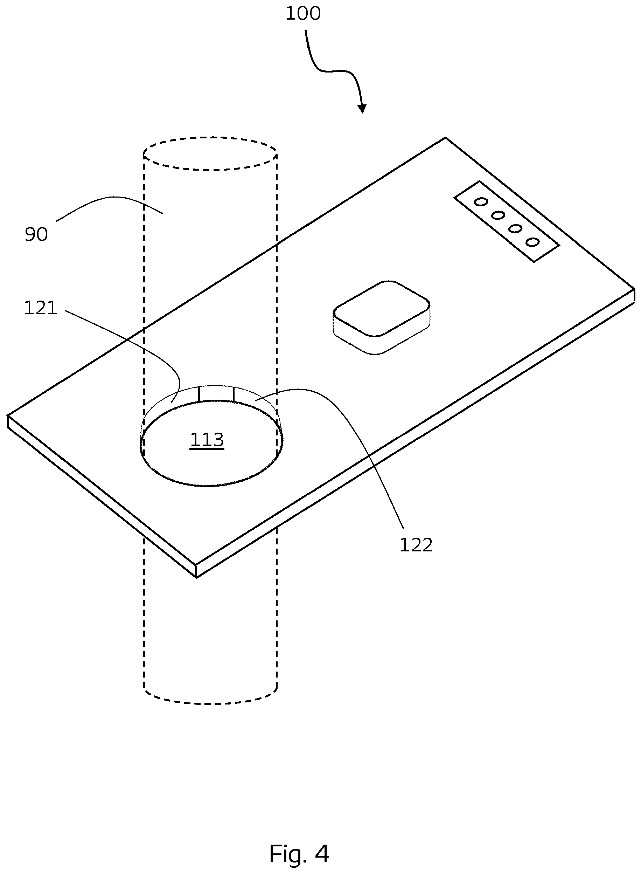

Fig. 4 illustrates a perspective view of an exemplary capacitive sensor assembly 100 according to an embodiment of the invention, such as the assembly 100 of Fig. 1, wherein a tubing 90, indicated by dashed lines, has been arranged in the passage 113. Thereby, a medium flowing through the tubing, such as a fluid or solid material, may be analysed by the capacitive sensor assembly 100. In particular, the assembly 100 may, such as when incorporated in and powered/controlled by a target device, measure one or more capacitive characteristics of the medium in the tubing 90 via the two-plate capacitor formed by the sensor face 121 and ground face 122 as discussed in relation to the foregoing figures. Further, the figure illustrates how the assembly 100 is non-invasive with regard to the tubing: the assembly 100, and in particular the passage 113, merely needs to be adapted to a size, such as a diameter, of the tubing 90, and does not require physical interaction with the medium in the tubing due to the underlying physics of a capacitive sensor.

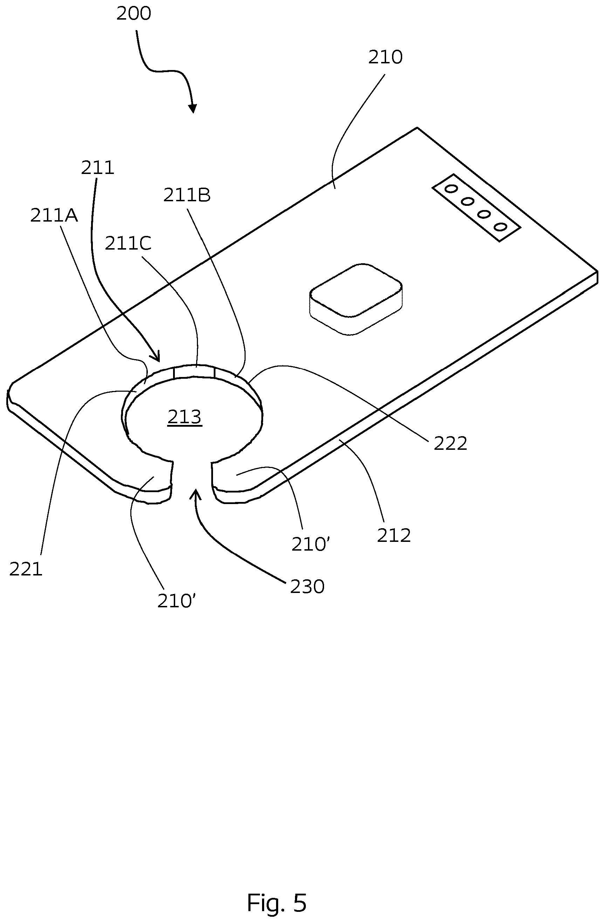

Fig. 5 illustrates a perspective view of an exemplary capacitive sensor assembly 200 according to an embodiment of the invention. The assembly 200 comprises, in addition to the like features discussed in the foregoing figures, a slit 230 connecting the interior edge 211 and the exterior edge 212. The slit 230 may be provided for manufacturing reasons, such as for faster or simpler manufacturing of the assembly 200, or the slit 230 may be provided to aid insertion of a tubing, such as where the neighbouring parts 210' forming the boundaries of the slit 230 are flexible. The overall shape of the passage 213 may remain circular as illustrated.

The slit 230 may be said to replace a non-conductive part of the interior edge 211 such that the interior edge 211 still comprises a conductive first part 211A and a conductive second part 211B, where the parts 211A,211B are thus separated by a non-conductive part 211C and the slit 230.

The first part 211A defines the sensor face 221 and the second part 211B defines the ground face 222, as explained in relation to the foregoing figures.

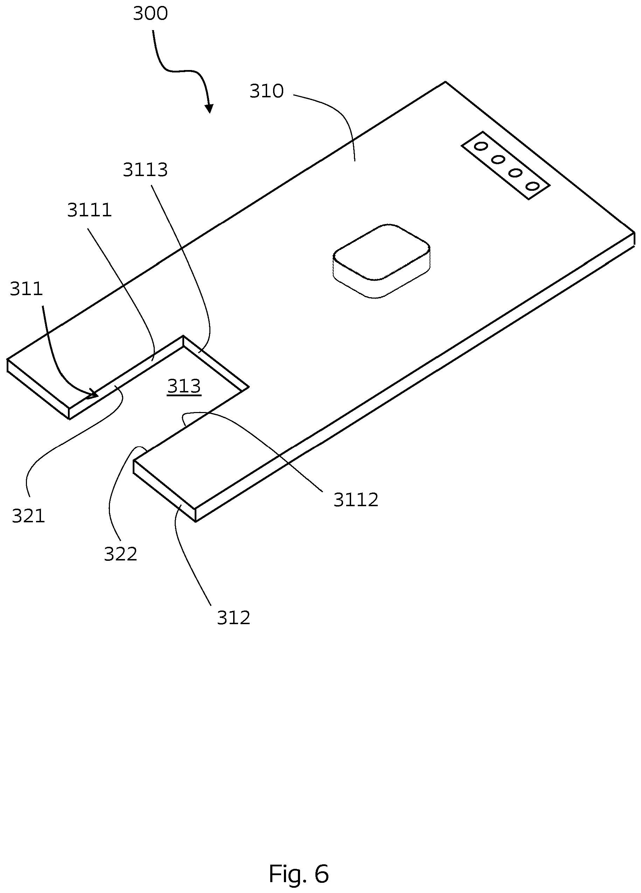

Fig. 6 illustrates a perspective view of an exemplary embodiment of a capacitive sensor assembly 300 according to an embodiment of the invention. In the embodiment, the passage 313 is embodied as a rectangular notch extending from the exterior edge 312 of the PCB 310 such that the interior edge 311 is connected to the exterior edge 312. In other words, the passage 313 comprises an opening to the surroundings in the assembly plane.

The interior edge 311 comprises three interior sub-edges configured to at least partly abut a tubing arranged in the notch. The three sub-edges include a first interior sub-edge 3111, a second interior sub-edge 3112 and a third interior sub-edge 3113. The first 3111 and the third sub-edge are connected and the second 3112 and the third sub-edge 3113 are connected. The second sub-edge 3112 is parallel with the first sub-edge 3111.

For example, a tubing may be pushed into contact with, and thus abut, the third sub-edge 3113 and partly abut/touch each of the first 3111 and second sub-edges 3112.

The first sub-edge 3111 is conductive in at least a portion of said edge, and the second sub-edge 3112 is conductive in at least a portion of said edge. The first sub-edge 3111 may define the sensor face 321 and the second sub-edge 3112 defines the ground face 322, thereby forming a two-plate capacitor.

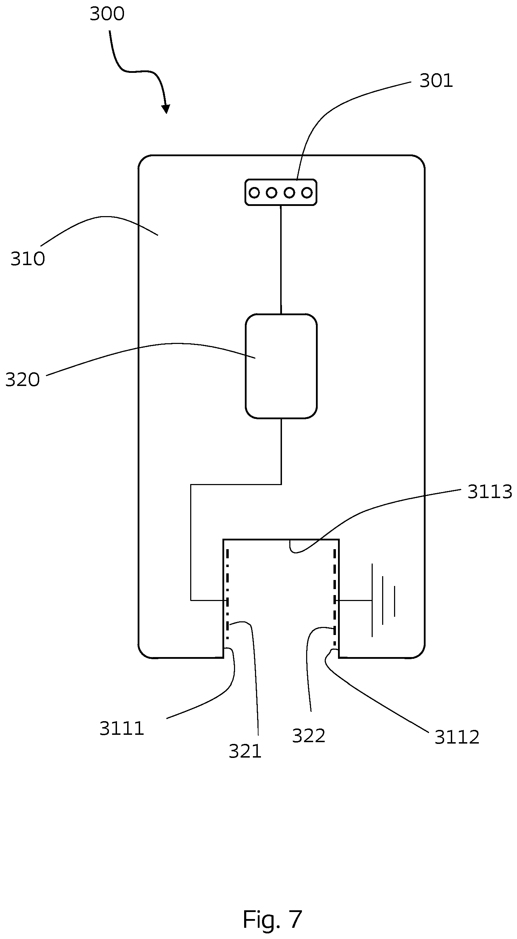

Fig. 7 illustrates a top view of an exemplary capacitive sensor assembly 300 according to an embodiment of the invention, such as the assembly 300 of Fig. 6. In the figure, the sensor face 321 is indicated by means of a dashed-dotted line parallel with the first sub-edge 3111 and the ground face 322 is indicated by means of a dashed line parallel with the second sub-edge 3112, and it is to be understood that the sensor face 321 and ground face 322 are thus arranged on respective parts, defined by the extent of the line/edge length, of the interior edge 311 of the PCB 300 partly defining the passage 313.

The length of the respective sensor face 321 and ground 322 may equal the length of the respective sub-edge 3111,3112, or the length may be less than the length of the respective sub-edge. The two faces 321,322 are separated by the non-conductive third sub-edge 3113. The ground face 322 is grounded, such as coupled to a ground terminal of the assembly interface 301. The sensor face 321 is connected to the module 320, which may further be connected to the assembly interface 301.

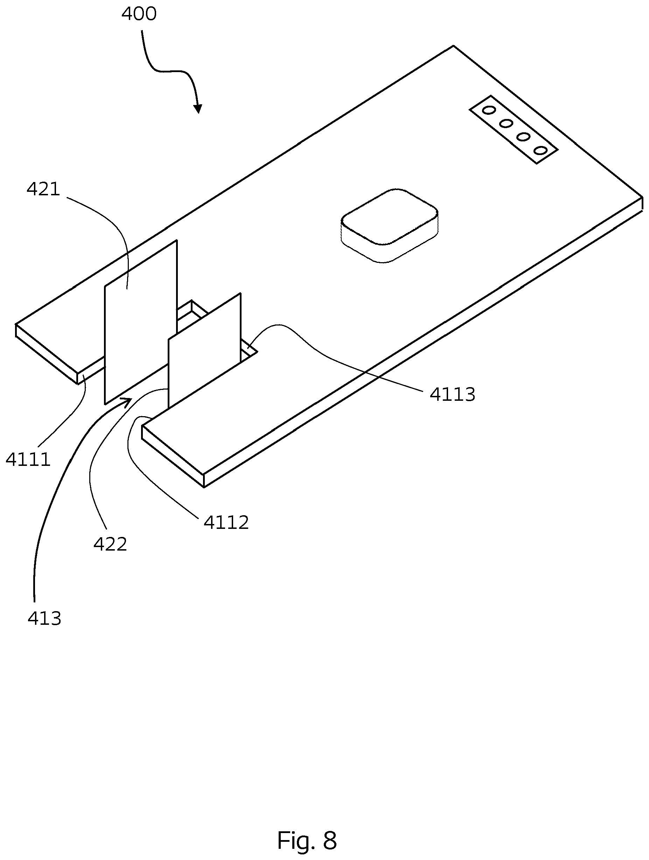

Fig. 8 illustrates a perspective view of an exemplary embodiment of a capacitive sensor assembly 400 according to an embodiment of the invention. The passage 413 is provided as a notch, similar to the embodiments discussed in relation to Figs. 6-7. In the present embodiment, the sensor face 421 and the ground face 422 are provided as conductive plates coupled to, such as soldered to, respective interior edges of the passage 413. In particular, the sensor face 421 is provided as a plate coupled to the first sub-edge 4111 and the ground face is provided as a plate coupled to the second sub-edge 4112. The plates serve to increase the surface area of the thus formed two-plate capacitor, which may increase the sensitivity of the capacitor. The plates may be planar, as illustrated, or the plates may be at least partly curved in accordance with an outer surface geometry/curvature of a tubing to be used with the assembly. For example, the plates may be curved in the vicinity of the respective edges closest to the third sub-edge 3113, such that a tubing may slide into the passage 413, i.e., into the notch, and abut the thus curved portions of the plates.

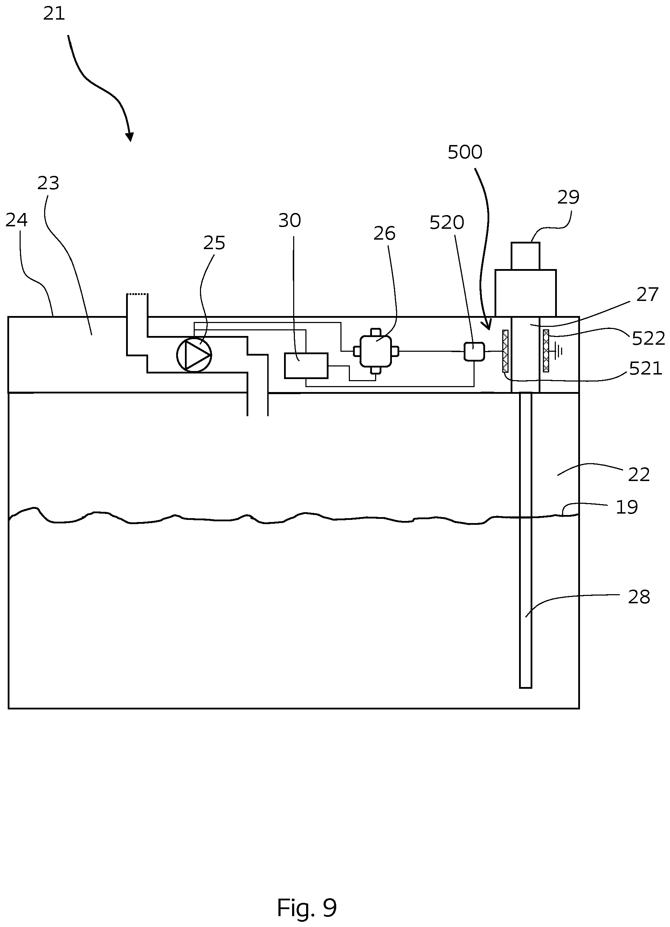

Fig. 9 illustrates a cross-sectional view of a part of an exemplary irrigation system comprising a capacitive sensor assembly 500 according to an embodiment of the invention. In particular, the figure illustrates a housing portion 21 of the irrigation system. The irrigation system may be considered an embodiment of a target device suitable for use with a capacitive sensor assembly as disclosed herein.

The housing 21 comprises a reservoir 22, wherein an irrigation liquid 19 is indicated, and an electronics compartment 23. In the illustrated embodiment, the electronics compartment 23 is arranged in a lid 24 of the housing 21. The electronics compartment 23 contains the majority of electronics associated with the irrigation system including a pump 25, at least one processor 26 and an intermediate tube section 27 connecting a dip tube 28 arranged in the reservoir 22 with a tube connector 29 configured to connect the housing with an irrigation tubing connected to a catheter or anal probe to be inserted in the rectum of a user. The pump 25 comprises an air intake. Preferably, the air intake comprises a filter. The pump 25 may be an air pump which comprises an air outlet in communication with the interior of the reservoir 22. Thereby, the air pump may pressurize the reservoir 22 by taking in ambient air via the air intake and outputting the air via the air outlet. The pressurization may force liquid into the dip tube 28 such that a flow of liquid is facilitated. The pump 25 is connected to the at least one processor 26 such that the at least one processor may control the pump, such as in accordance with the one or more capacitive characteristics as measured by the capacitive sensor of the assembly 500.

The assembly 500 is arranged in the electronics compartment 23, and in particular, the assembly 500 is arranged such that the capacitive sensor of the assembly 500 may measure one or more capacitive characteristics of a fluid in the intermediate tube section 27. The assembly 500 is drawn schematically but generally comprises the sensor face 521, the ground face 522, and the capacitive sensor module 520 connected (such as via an assembly interface not explicitly illustrated) to the processor 26 and the power unit 30 of the irrigation system. For example, the sensor and ground faces are exaggerated to highlight their arrangement relative to the intermediate tube section 27, which thus constitutes the tubing 90 as referred to in previous embodiments. Preferably, the ground face 522 is a floating ground grounded relative to the power unit (battery) 30.

The one or more capacitive characteristics may be an absolute value of the capacitance or permittivity, or the one or more capacitive characteristics may be an indication of whether the capacitance or permittivity is above or below one or more thresholds, such that the assembly 500 may provide a binary output indicative of whether the intermediate tube section 27 comprises a liquid or a gas. The assembly 500 is connected to the processor 26 and powered by a power unit 30 of the irrigation system 20.

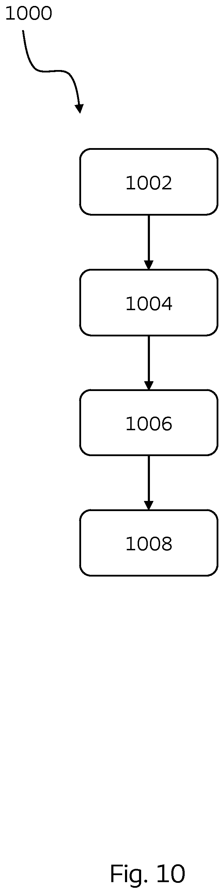

Fig. 10 illustrates a method 1000 of manufacturing an irrigation system according to embodiments herein. The method comprises the steps of:
- providing 1002 the housing, the tubing, the control unit, and the pump;
- providing 1004 the capacitive sensor assembly;
- assembling 1006 the housing, the control unit, the pump, and the tubing; and
- arranging 1008 the capacitive sensor assembly relative to the tubing such that the tubing passes through the passage of the capacitive sensor assembly.

The method 1000 highlights a benefit of the capacitive sensor assembly: namely, the capacitive sensor assembly may be provided as one-piece in the manufacturing line of the irrigation system. Thereby, the irrigation system may be manufactured through providing the individual pieces and assembling the pieces, wherein the step of arranging the capacitive sensor assembly relative to the tubing involves arranging the assembly such that it is configured to measure one or more capacitive characteristics of a fluid in the tubing. This is achieved by means of arranging the tubing in the passage of the assembly. Namely, the sensor face and the ground face are configured, and thus arranged, to measure the one or more characteristics of a medium (here, fluid) in the tubing when arranged in the passage.

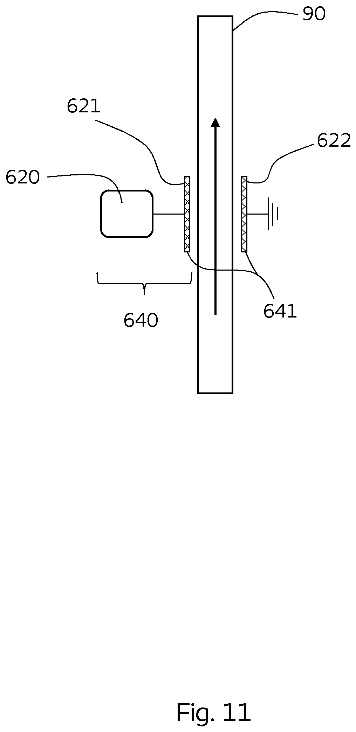

Fig. 11 illustrates an exemplary build of a capacitive sensor 640 of the capacitive sensor assembly according to embodiments not part of the claimed invention. Also illustrated is a tubing 90 extending through the capacitor 641 formed by the sensor face 621 and the ground face 622. The ground face 622 is grounded and the sensor face 621 is coupled to the capacitive proximity sensor module 620.

In the illustrated embodiment, a parallel-plate capacitor 641 is formed, whereby the theory of such a capacitor may be used to qualitatively discuss the physics.

The capacitive sensor 640 is configured to monitor one or more capacitive characteristics, such as capacitance C or permittivity *ε*, over time: C(t) is dependent on the changes of permittivity (of the medium in the tubing) over time *ε*(t) in the capacitor: $C \left(t\right) = ε \left(t\right) \frac{A}{d} = {ε}_{r} \left(t\right) {ε}_{0} \frac{A}{d}$ wherein *ε* is the permittivity of the (dielectric) medium between the parallel plates, A is the area of each of the parallel plates, and d is the separation of the parallel plates, said separation being a gap filled with the (dielectric) medium. The permittivity *ε* may be expressed as the product of the relative permittivity *ε*ᵣ of the medium and the vacuum permittivity *ε*₀. As such, a medium having a permittivity *ε* may be said to induce a capacitance when the area A and the separation d are constant. The vacuum permittivity *ε*₀ is a physical constant having the value 8.8541878128 × 10⁻¹² F m⁻¹, and the relative permittivity *ε*ᵣ of water is 80.2 (at 20 °C) and the relative permittivity of air is 1.00058986. For vacuum, the relative permittivity is one by definition. Thus, the relative permittivity of water is approximately 80 times the relative permittivity of air, which translates to a similar change of the size of the capacitance of the capacitive sensor when a change from liquid to air occurs in the tubing in proximity to the capacitive sensor and vice versa.

Accordingly, the assembly, or the capacitive sensor thereof, is configured to monitor the time-dependent capacitive characteristics, such that the assembly may monitor changes in the capacitive characteristic and preferably generate a signal, such as a binary output, indicative of such change, such as a change from the first capacitance to a second capacitance different from the first capacitance.

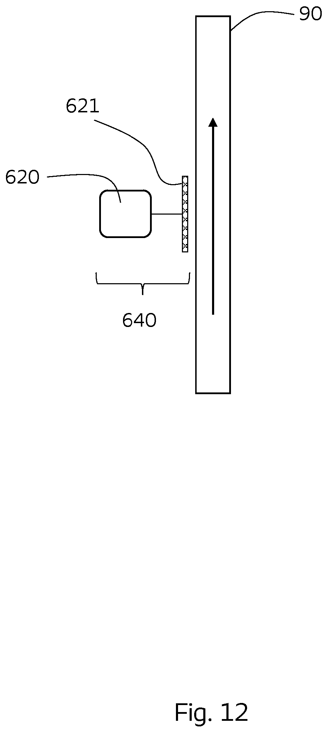

Fig. 12 illustrates an exemplary build of a capacitive sensor 740 of the capacitive sensor assembly according to embodiments not part of the claimed invention. The capacitive sensor 740 may be considered an alternative to the build of Fig. 11. In Fig. 12, the ground face is omitted, such that the capacitive sensor 740 solely comprises the sensor face 721 and the module 720, where the sensor face 721 is arranged to abut the tubing 90. In such an embodiment, the capacitive sensor 740 may be considered a proximity or distance sensor, and whereas it may be more prone to external disturbances, it may be shielded by other means, such as by means of shielding the target device. It is appreciated that embodiments of the assembly as discussed in relation to Figs. 1-8 may all be fitted with a capacitive sensor 740 according to the embodiment of Fig. 12. For example, previous embodiments of the assembly may be provided with just a sensor face 721 merely by omitting the ground face from the builds/embodiments.

The assembly as disclosed herein may comprise further auxiliary electronic components required for acquiring the one or more capacitive characteristics, but which auxiliary electronic components are commonly employed to capacitive circuits in the field of electronics, such as internal capacitors for the circuit, resistors in the circuitry, and the like. Likewise, the module may comprise a memory and/or a power unit, in addition to a processor as disclosed, without departing from the scope of the invention.

Whereas an irrigation system has been disclosed as a specific embodiment of a target device for use with the capacitive sensor assembly as disclosed herein, it is appreciated that the assembly may be used in a wide range of applications, including, but not limited to, monitoring of gas pipes, monitoring of water pipes, monitoring of pipes in a manufacturing facility where objects are passing through a tubing, and where it is desired to monitor the speed, size, number or the like of such objects. For example, the objects may be counted by means of analysing the change of capacitance or permittivity over time, as the passing of an object alters the capacitance if the permittivity of the object is different from, e.g., the ambient air in the tubing. Further, the assembly may be included in medical devices wherein fluids are flowing, such as where it is desired to know whether liquid or gas is present in the tubing, such as in dialysis, during infusion, catheterization, or bowel irrigation.

The use of the terms "first", "second", "third" and "fourth", "primary", "secondary", "tertiary" etc. does not imply any particular order but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering.

Furthermore, the labelling of a first element does not imply the presence of a second element and vice versa.

It is to be noted that the words "comprising" and "including" do not necessarily exclude the presence of other elements or steps than those listed.

It is to be noted that the words "a" or "an" preceding an element or method step do not exclude the presence of a plurality of such elements or method steps.

It should further be noted that any reference signs do not limit the scope of the claims, that the exemplary embodiments may be implemented at least in part by means of both hardware and software, and that several "means", "units" or "devices" may be represented by the same item of hardware.

The various exemplary methods, devices, and systems described herein are described in the general context of method steps processes or actions by a system or processor, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program modules may include routines, programs, objects, components, data structures, etc. that perform specified tasks or implement specific abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes.

Although particular features have been shown and described, it will be understood that they are not intended to limit the claimed invention, and it will be made obvious to those skilled in the art that various changes and modifications may be made without departing from the scope of the claimed invention. Accordingly, the specification and drawings are to be regarded in an illustrative rather than restrictive sense. The claimed invention is intended to cover all alternatives, modifications, and equivalents.

## Claims

1. An irrigation system for bowel irrigation comprising a capacitive sensor assembly (500) for measuring one or more capacitive characteristics of a fluid in a tubing (90, 27) of the irrigation system, the irrigation system comprising:
- a housing (21) comprising a reservoir (22) for containing an irrigation liquid (19);
- the tubing (90, 27), providing fluid communication between the reservoir (22) and a catheter for use with the irrigation system;
- a control unit comprising a processor (26) coupled to the capacitive sensor assembly (500);
- a pump (25) for facilitating a flow of irrigation liquid from the reservoir (22), through the tubing (90, 27), to the catheter; and
- the capacitive sensor assembly (500) comprising an assembly interface (101) for connection to the irrigation system and further comprising a printed circuit board (PCB) comprising an interior edge (111, 211, 311, 4111, 4113, 4111) defining a passage (113, 213, 313, 413) for the tubing (90, 27), the interior edge being configured to at least partly abut the tubing (90, 27); a capacitive proximity sensor module (520); a conductive sensor face (521) coupled to the capacitive proximity sensor module (520); and a conductive ground face (522) electrically grounded relative to the capacitive proximity sensor module (520) and arranged facing the sensor face (521) to form a two-plate capacitor; wherein the capacitive proximity sensor module (520), the sensor face (521), and the ground face (522) are arranged on the PCB and configured to measure the one or more capacitive characteristics of a fluid in the tubing (90, 27);
wherein the tubing (90, 27) is arranged in the passage of the PCB of the capacitive sensor assembly (500).

2. The irrigation system according to claim 1, wherein the interior edge is electrically conducting in a first part and in a second part separate from the first part.

3. The irrigation system according to claim 2, wherein the first part of the interior edge forms the sensor face, and wherein the second part of the interior edge forms the ground face.

4. The irrigation system according to any of claims 1-3, wherein the passage is a through-going opening in the PCB.

5. The irrigation system according to claim 4, wherein the through-going opening is circular.

6. The irrigation system according to claim 5, wherein the sensor face and the ground face are semicylindrical faces on the interior edge.

7. The irrigation system according to any of claims 4-6 as dependent on claim 2, wherein each of the first part and the second part of the interior edge is configured to abut the tubing in an arc less than 170°.

8. The irrigation system according to any of claims 1-3, wherein the passage is a notch extending from an exterior edge of the PCB and wherein the interior edge is connected to the exterior edge.

9. The irrigation system according to claim 8, wherein the interior edge comprises three interior sub-edges configured to at least partly abut the tubing, the three interior sub-edges comprising a first interior sub-edge and a second interior sub-edge, the second interior sub-edge being arranged in parallel with, and separate from, the first interior sub-edge.

10. The irrigation system according to claim 9, wherein the first interior sub-edge is conductive and defines the sensor face and the second interior sub-edge is conductive and defines the ground face.

11. The irrigation system according to any of claims 1-11, wherein the PCB is planar and has a first thickness of maximum 3 mm.

12. A method (1000) of manufacturing an irrigation system according to any of claims 1-11, the method comprising the steps of:
- providing (1002) the housing, the tubing, the control unit and the pump;
- providing (1004) the capacitive sensor assembly;
- assembling (1006) the housing, the control unit, the pump and the tubing; and
- arranging (1008) the capacitive sensor assembly relative to the tubing such that said capacitive sensor assembly is configured to measure one or more capacitive characteristics of a fluid in the tubing.

## Patentansprüche

1. Spülsystem zur Darmspülung, umfassend eine kapazitive Sensoranordnung (500) zum Messen einer oder mehrerer kapazitiver Eigenschaften eines Fluids in einem Schlauch (90, 27) des Spülsystems, wobei das Spülsystem umfasst:
- ein Gehäuse (21), das einen Behälter (22) zur Aufnahme einer Spülflüssigkeit (19) umfasst;
- den Schlauch (90, 27), der eine Fluidverbindung zwischen dem Behälter (22) und einem Katheter zur Verwendung mit dem Spülsystem bereitstellt;
- eine Steuereinheit, die einen Prozessor (26) umfasst, der mit der kapazitiven Sensoranordnung (500) gekoppelt ist;
- eine Pumpe (25) zur Erleichterung eines Flusses von Spülflüssigkeit aus dem Behälter (22) durch den Schlauch (90, 27) zum Katheter; und
- wobei die kapazitive Sensoranordnung (500) eine Anordnungsschnittstelle (101) zur Verbindung mit dem Spülsystem umfasst und ferner eine Leiterplatte (PCB) umfasst, die einen Innenrand (111, 211, 311, 4111, 4113, 4111) umfasst, der einen Durchgang (113, 213, 313, 413) für den Schlauch (90, 27) definiert, wobei der Innenrand dazu ausgelegt ist, zumindest teilweise an den Schlauch (90, 27) anzustoßen; ein kapazitives Näherungssensormodul (520); eine leitfähige Sensorfläche (521), die mit dem kapazitiven Näherungssensormodul (520) gekoppelt ist; und eine leitfähige Massefläche (522), die relativ zu dem kapazitiven Näherungssensormodul (520) elektrisch geerdet ist und der Sensorfläche (521) zugewandt angeordnet ist, um einen Zweiplattenkondensator zu bilden; wobei das kapazitive Näherungssensormodul (520), die Sensorfläche (521) und die Massefläche (522) auf der PCB angeordnet und dazu ausgelegt sind, die eine oder die mehreren kapazitiven Eigenschaften eines Fluids in dem Schlauch (90, 27) zu messen;
wobei der Schlauch (90, 27) in dem Durchgang der PCB der kapazitiven Sensoranordnung (500) angeordnet ist.

2. Spülsystem nach Anspruch 1, wobei der Innenrand in einem ersten Teil und in einem zweiten Teil, der von dem ersten Teil getrennt ist, elektrisch leitend ist.

3. Spülsystem nach Anspruch 2, wobei der erste Teil des Innenrandes die Sensorfläche bildet und wobei der zweite Teil des Innenrandes die Massefläche bildet.

4. Spülsystem nach einem der Ansprüche 1-3, wobei der Durchgang eine durchgehende Öffnung in der PCB ist.

5. Spülsystem nach Anspruch 4, wobei die durchgehende Öffnung kreisförmig ist.

6. Spülsystem nach Anspruch 5, wobei die Sensorfläche und die Massefläche halbzylindrische Flächen an dem Innenrand sind.

7. Spülsystem nach einem der Ansprüche 4-6 in Abhängigkeit von Anspruch 2, wobei sowohl der erste Teil als auch der zweite Teil des Innenrandes dazu ausgelegt sind, in einem Bogen von weniger als 170° an dem Schlauch anzustoßen.

8. Spülsystem nach einem der Ansprüche 1-3, wobei der Durchgang eine Kerbe ist, die sich von einem Außenrand der PCB erstreckt, und wobei der Innenrand mit dem Außenrand verbunden ist.

9. Spülsystem nach Anspruch 8, wobei der Innenrand drei innere Unterränder umfasst, die dazu ausgelegt sind, zumindest teilweise an den Schlauch anzustoßen, wobei die drei inneren Unterränder einen ersten inneren Unterrand und einen zweiten inneren Unterrand umfassen, wobei der zweite innere Unterrand parallel zu und getrennt von dem ersten inneren Unterrand angeordnet ist.

10. Spülsystem nach Anspruch 9, wobei der erste innere Unterrand leitfähig ist und die Sensorfläche definiert und der zweite innere Unterrand leitfähig ist und die Massefläche definiert.

11. Spülsystem nach einem der Ansprüche 1-11, wobei die PCB planar ist und eine erste Dicke von maximal 3 mm aufweist.

12. Verfahren (1000) zum Fertigen eines Spülsystems nach einem der Ansprüche 1-11, wobei das Verfahren die folgenden Schritte umfasst:
- Bereitstellen (1002) des Gehäuses, des Schlauchs, der Steuereinheit und der Pumpe;
- Bereitstellen (1004) der kapazitiven Sensoranordnung;
- Zusammenbauen (1006) des Gehäuses, der Steuereinheit, der Pumpe und des Schlauchs; und
- Anordnen (1008) der kapazitiven Sensoranordnung relativ zu dem Schlauch, so dass die kapazitive Sensoranordnung dazu ausgelegt ist, eine oder mehrere kapazitive Eigenschaften eines Fluids in dem Schlauch zu messen.

## Revendications

1. Système d'irrigation pour l'irrigation intestinale comprenant un ensemble capteur capacitif (500) pour mesurer une ou plusieurs caractéristiques capacitives d'un fluide dans une tubulure (90, 27) du système d'irrigation, le système d'irrigation comprenant :
- un logement (21) comprenant un réservoir (22) pour contenir un liquide d'irrigation (19) ;
- la tubulure (90, 27), assurant une communication fluidique entre le réservoir (22) et un cathéter destiné à être utilisé avec le système d'irrigation ;
- une unité de commande comprenant un processeur (26) couplé à l'ensemble capteur capacitif (500) ;
- une pompe (25) pour faciliter un écoulement du liquide d'irrigation depuis le réservoir (22), à travers la tubulure (90, 27), jusqu'au cathéter ; et
- l'ensemble capteur capacitif (500) comprenant une interface d'ensemble (101) pour une connexion au système d'irrigation et comprenant en outre une carte de circuit imprimé (PCB) comprenant un bord intérieur (111, 211, 311, 4111, 4113, 4111) définissant un passage (113, 213, 313, 413) pour la tubulure (90, 27), le bord intérieur étant configuré pour venir en butée au moins en partie contre la tubulure (90, 27) ; un module de capteur de proximité capacitif (520) ; une face de capteur conducteur (521) couplée au module de capteur de proximité capacitif (520) ; et une face de masse conductrice (522) électriquement reliée à la masse par rapport au module de capteur de proximité capacitif (520) et agencée en regard de la face de capteur (521) pour former un condensateur à deux plaques ; dans lequel le module de capteur de proximité capacitif (520), la face de capteur (521) et la face à la masse (522) sont agencés sur la PCB et configurés pour mesurer la ou les caractéristiques capacitives d'un fluide dans la tubulure (90, 27) ;
dans lequel la tubulure (90, 27) est agencée dans le passage de la PCB de l'ensemble capteur capacitif (500).

2. Système d'irrigation selon la revendication 1, dans lequel le bord intérieur est électroconducteur dans une première partie et dans une seconde partie séparée de la première partie.

3. Système d'irrigation selon la revendication 2, dans lequel la première partie du bord intérieur forme la face de capteur, et dans lequel la seconde partie du bord intérieur forme la face à la masse.

4. Système d'irrigation selon l'une quelconque des revendications 1 à 3, dans lequel le passage est une ouverture traversante dans la PCB.

5. Système d'irrigation selon la revendication 4, dans lequel l'ouverture traversante est circulaire.

6. Système d'irrigation selon la revendication 5, dans lequel la face de capteur et la face à la masse sont des faces semi-cylindriques sur le bord intérieur.

7. Système d'irrigation selon l'une quelconque des revendications 4 à 6 lorsqu'elles dépendent de la revendication 2, dans lequel chacune de la première partie et de la seconde partie du bord intérieur est configurée pour venir buter contre le tube selon un arc inférieur à 170°.

8. Système d'irrigation selon l'une quelconque des revendications 1 à 3, dans lequel le passage est une encoche s'étendant à partir d'un bord extérieur de la PCB et dans lequel le bord intérieur est connecté au bord extérieur.

9. Système d'irrigation selon la revendication 8, dans lequel le bord intérieur comprend trois sous-bords intérieurs configurés pour venir au moins partiellement en butée contre la tubulure, les trois sous-bords intérieurs comprenant un premier sous-bord intérieur et un second sous-bord intérieur, le second sous-bord intérieur étant agencé parallèlement au premier sous-bord intérieur et séparé de celui-ci.

10. Système d'irrigation selon la revendication 9, dans lequel le premier sous-bord intérieur est conducteur et définit la face de capteur et le second sous-bord intérieur est conducteur et définit la face à la masse.

11. Système d'irrigation selon l'une quelconque des revendications 1 à 11, dans lequel la PCB est plane et a une première épaisseur de 3 mm maximum.

12. Procédé (1000) de fabrication d'un système d'irrigation selon l'une quelconque des revendications 1 à 11, le procédé comprenant les étapes suivantes :
- fournir (1002) le boîtier, la tubulure, l'unité de contrôle et la pompe ;
- fournir (1004) l'ensemble capteur capacitif ;
- assembler (1006) le boîtier, l'unité de commande, la pompe et la tubulure ; et
- agencer (1008) l'ensemble capteur capacitif par rapport à la tubulure de telle sorte que ledit ensemble capteur capacitif soit configuré pour mesurer une ou plusieurs caractéristiques capacitives d'un fluide dans la tubulure.
